# EUROPEAN PATENT APPLICATION

(11) **EP 4 563 167 A1**
(43) Date of publication of application: **04.06.2025**
(21) Application number: 24383290.4
(22) Date of filing: 28.11.2024
(51) Int. Cl.: A61L 9/01, A61L 9/04, A61L 9/12, A61L 9/14, A45D 34/02

(54) **LIQUID PERFUME FORMULATION FOR USE IN NEBULIZING DIFUSSERS AND OR EVAPORATIVE DIFUSSERS**

(30) Priority: 28.11.2023 ES 202330986
(71) Applicant: Vila Hermanos Cereria, S.A., 46869 Adzaneta De Albaida Valencia (ES)
(72) Inventor: GUY DOUENAT, Patrick, 46869 Adzaneta de Albaida (Valencia) (ES)
(74) Representative: Ungria López, Javier

(57) **Abstract**

The present invention relates to a liquid perfume formulation comprising at least one essence, in such a percentage that the percentage of volatile organic compounds provided by the essence to the formulation is equal to or less than 18% by weight; at least one alcohol, in such a percentage that the sum of the percentage of volatile organic compounds provided by the essence and the alcohol to the formulation is equal to or less than 18% by weight; and decamethylcyclopentasiloxane, in the required percentage up to 100% by weight. Another object of the invention is the use of the formulation in a liquid perfume dispenser and the liquid perfume dispenser characterised in that it comprises a reservoir open to the atmosphere and containing said perfume formulation.

## Description

### TECHNICAL SECTOR

The object of the invention belongs to the chemical industry sector. In particular, it relates to a novel liquid perfume formulation for use in nebulizing diffusers and/or natural evaporative diffusers.

### VOC

### BACKGROUND OF THE INVENTION

Although there are different versions of perfume or fragrance diffusers, one of the most widespread types is the reed diffuser (Mikado type). This diffuser consists of a reservoir open to the atmosphere by means of a mouthpiece in which a series of reeds, usually made of wood, but which can be made of any rigid porous material, are included. These reeds are partly immersed in the perfume and partly in contact with the atmosphere. The perfume is absorbed by the reeds by capillarity and, at the end in contact with the atmosphere, it evaporates and transfers to said atmosphere, providing a long-lasting perfume without the need for other means.

Such devices require very little maintenance, where the reeds only need to be rotated occasionally to maintain the scent and, once the perfume has been used up, the reservoir only needs to be refilled to maintain its function.

In devices of this type, typically the formulation of the perfume, in liquid state, often contains components that aid evaporation due to their low boiling point. Nevertheless, in its design, a balance between the intensity of the fragrance and the duration of said fragrance has to be achieved since, if highly volatile components are used, the intensity of the fragrance is very high, but its durability is drastically reduced.

Currently, in the formulations used for the perfumes of evaporative diffusers of this type, alcohol is widely used as the base in which the essence is dissolved, said essence being the precursor of the aroma. Said base may comprise other additional components, such as additives suitable for providing the blend with colour, greater resistance to evaporation or better diffusion of the perfume, among other properties.

Alcohol is a fairly widespread solvent base because it has suitable characteristics in terms of essence integration and evaporation, while maintaining a very competitive economic cost. The main improvements of alcohol-based formulations are primarily aimed at slowing evaporation, maintaining the perfume for more time, and not substantially diminishing the aroma.

Alternative formulations include bases with greater resistance to evaporation. However, these solvents tend to be more expensive and more difficult to obtain. Likewise, these alternative solvents do not have good aroma diffusion and, therefore, require a larger amount of essence to achieve a similar effect. As such, they are often used in combination with alcohol to generate blends with improved characteristics.

Despite the widespread use of alcohol in evaporable fragrance formulations, its use as a base for said formulations entails a number of drawbacks and limitations. Alcohol, especially in the concentration in which it is usually used, is highly flammable. Moreover, it also contains a large amount of volatile organic compounds (VOCs), which are released during the evaporation process of the fragrance.

As a result of the foregoing limitations, countries have developed very restrictive legislation concerning the import of alcohol-based perfume formulations, which makes it difficult to export them abroad. In particular, there are countries such as the USA or Canada that have imposed such strict limits that formulations used domestically (in Spain) or in Europe cannot be exported to said countries. For this reason, specific formulations using alternative solvents to alcohol, which are much less cost-effective, have been developed in recent years, which has increased the cost of the final product. This fact, along with the increase in the price of transportation, has meant that exporting such products to non-European countries is currently far from competitive, which has limited the outsourcing of the sector to other parts of the world.

Another drawback associated with the use of alcohol-based formulations is that they do not allow the use of coloured reeds in the diffuser devices, as alcohol is a strong solvent that will eventually affect the colour. An additional limitation associated with this fact is the damage that may be caused by possible spillage of the formulation, for example, during transport thereof, or when replacing the perfume or rotating the reeds in the device. Likewise, it may sometimes happen that part of the evaporated components condense and fall on the surface where the device is located, producing stains on said surface.

In order to overcome the aforementioned limitations, a novel formulation has been developed with improved characteristics compared to existing formulations, substantially reducing the content of VOCs, but maintaining and even improving the diffusion capacity of the fragrances or aromas contained in the formulation. Due to the reduction in the percentage of VOCs, the developed formula can be exported to any country in the world by avoiding the strict regulations of certain countries with respect to the permitted VOC limits.

### DESCRIPTION OF THE INVENTION

In this regard, a first object of the invention is a liquid perfume formulation characterised in that it comprises a percentage of volatile organic compounds (VOC) equal to or less than 18% by weight. To determine the amount of VOCs in the formulation, any existing measurement method can be used, such as those consisting of a sampling unit, a unit for pre-concentration/desorption/injection of the VOCs contained in the sampling unit, a chromatography column, a flame ionisation detector (FID) and/or a mass spectrometer (C. Puente, R. Ramaroson, Vol. 19 No. 1 (2006): Revista ION).

For the purposes of the present patent application, volatile organic compound (VOC) is understood to be an organic compound that, at 20°C, has a vapour pressure equal to or greater than 0.01 kPa.

Particularly, the formulation object of the invention is characterised in that it comprises a blend of at least one essence, at least one alcohol (preferably ethanol and more preferably isopropyl alcohol, for its volatility and low odour) and decamethylcyclopentasiloxane as a solvent, which adds volatility to the perfume.

For the purposes of the present patent application, essence is understood to be the odorous substance contained in a volatile liquid which is not very soluble in water. In particular embodiments of the invention, the essence may be contained in an essential oil obtained from the extraction of aromatic plants such as jasmine, mint or lavender, therefore being a natural essence. In other embodiments, it may consist of a synthetic essence. The combination of essences in the formulation gives rise to the aroma or fragrance of the perfume.

In turn, the presence of alcohol and preferably isopropyl alcohol in the formulation helps to trigger the evaporation reaction and improves the diffusion capacity of the perfume. In a particular embodiment of the invention, isopropyl alcohol can be from a renewable source, which contributes to providing an environmentally friendly formulation.

Of the three components identified, the essence is the one with the highest percentage of VOCs. In this regard, the percentage of alcohol and preferably of isopropyl alcohol in the formulation will be a function of the percentage of essence and, in particular, of the percentage of VOCs in the essence, such that the final percentage of VOCs in the formulation will always be equal to or less than 18% by weight.

Thus, in a particular embodiment in which 100% of the essence is VOCs and the percentage of essence is 15% by weight, the percentage of isopropyl alcohol will be equal to or less than 3% by weight.

In a general manner, the claimed perfume formulation may comprise, as a percentage by weight of the total:
· at least one essence, in such a percentage that the percentage of VOCs provided by the essence to the formulation is equal to or less than 18% by weight. In particular embodiments of the invention, the percentage of essence in the formulation may be between 2% and 25% by weight, and more preferably between 5% and 10% by weight;
· at least one alcohol, preferably ethanol and more preferably isopropyl alcohol, which specific percentage in the formulation will be adjusted based on the percentage of VOCs provided by the essence, such that the sum of the percentage of VOCs provided by the essence and the alcohol is always equal to or less than 18% by weight. In particular embodiments of the invention, the percentage of alcohol in the formulation may be equal to or less than 16% by weight, possibly reaching values close to zero or even not being present in the formulation. Preferably, the percentage of alcohol in the formulation will be between 1% and 10% by weight; and
· decamethylcyclopentasiloxane, in the required percentage up to 100% by weight.

In particular embodiments of the invention, the claimed perfume formulation may additionally comprise at least one additive, in which case the percentage of decamethylcyclopentasiloxane will be adjusted, such that the sum of the same with the additive(s) added to the formulation, taking into account the percentage of essence and isopropyl alcohol, results in 100% by weight of the formulation.

In a particular embodiment of the invention, the additive may be a dye, preferably in a percentage equal to or less than 1% by weight.

The claimed formulation has been shown to reduce the percentage of VOCs and, at the same time, maintain a constant evaporation curve of the perfume over time, similar to that of the perfume formulations currently available in the state of the art.

Another object of the invention is the use of the claimed perfume formulation in a liquid perfume dispenser, preferably in a Mikado-type perfume dispenser or diffuser and/or in a nebulizing diffuser, which is preferably electric.

Additionally, the object of the invention is a perfume dispenser comprising a reservoir open to the atmosphere and containing a perfume formulation as claimed, as well as at least one reed extending from a first end in contact with the liquid perfume formulation contained in the reservoir to a second end in contact with the atmosphere, from where the perfume formulation evaporates and disperses into the atmosphere surrounding the dispenser.

### BRIEF DESCRIPTION OF THE FIGURES

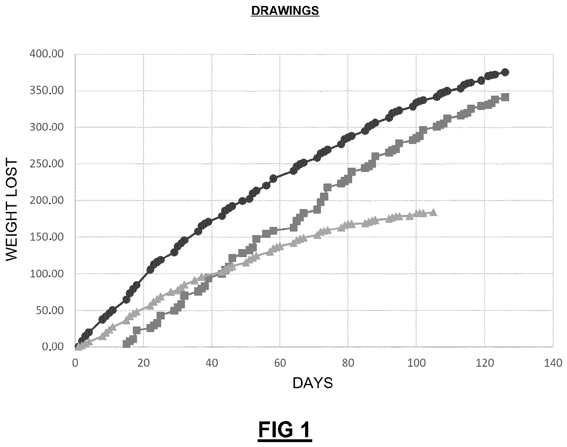

To complement the description that is being made and for the purpose of helping to better understand the features of the invention, Figure 1 is included which, in an illustrative and non-limiting manner, shows an evaporation curve of the claimed formulation over time (days) in comparison with two alternative formulations of the state of the art.

### PREFERRED DESCRIPTION OF THE INVENTION

In order to test the evaporation process of the claimed perfume formulation in comparison with that of other perfume formulations available in the state of the art, a comparative study of the evaporation curves of two known perfume formulations versus the evaporation curve of a perfume formulation according to the object of the present invention was carried out.

In particular, a perfume formulation was prepared according to the following composition:
· 72% by weight of decamethylcyclopentasiloxane;
· 18% by weight of essence; and
· 10% by weight of isopropyl alcohol.

This formulation was compared with two commercial formulations, in particular, Disonol^{®} (traditional formulation) and Augeo^{®}, which have the compositions shown in Table 1 and wherein percentages are percentages by weight:

**Table 1**

| **Disonol^{®}** | **Augeo^{®}** |
|---|---|
| 74% Disonol (95% ethanol and 5% isopropyl alcohol) | |
| 8% Augeo* | 82% Augeo |
| 18% essence | 18% essence |

| | |
|---|---|
| * Augeo refers to the trade name of a slow-evaporating glycerine solvent from a renewable source, which is one of the most widespread alternatives on the market for use in air fresheners and perfumes. This is the closest alternative to the object of the invention, as it does not contain VOCs in a percentage greater than 18% by weight. | |

The obtained results are shown in Figure 1, where it can be seen that the Disonol^{®} formulation (•) has the best evaporation curve, although the curve for the claimed perfume formulation (▪) is higher than the curve for the Augeo^{®} formulation (A). As can also be seen in Figure 1, Augeo^{®} loses its capacity to evaporate before the rest of the compositions. Likewise, as its evaporation capacity is lower than that of the other formulations, it is a product with a lower olfactory capacity.

The traditional formulation, Disonol^{®}, has the best evaporation curve, maintaining an almost constant evaporation capacity over time. Nevertheless, as discussed above, this formulation has the problem that it contains VOCs in a concentration that is much higher than what is allowed in some countries, which limits the possibility of selling and exporting it worldwide and, furthermore, it can lead to environmental problems in the event of intensive use.

Lastly, as shown in Figure 1, although the claimed formulation has a lower evaporation capacity than the traditional formulation (Disonol^{®}), it maintains an almost constant evaporation capacity and has better characteristics in this regard than the substitute formulations on the market (Augeo^{®}).

## Claims

1. A liquid perfume formulation **characterised in that** it comprises:
· at least one essence, in such a percentage that the percentage of volatile organic compounds provided by the essence to the formulation is equal to or less than 18% by weight;
· at least one alcohol, in such a percentage that the sum of the percentage of volatile organic compounds provided by the essence and the alcohol to the formulation is equal to or less than 18% by weight; and
· decamethylcyclopentasiloxane, in the required percentage up to 100% by weight;
wherein the percentage of volatile organic compounds in the formulation is equal to or less than 18% by weight.

2. The perfume formulation according to claim 1, wherein the alcohol is ethanol or isopropyl alcohol.

3. The perfume formulation according to claim 1 or 2, wherein the percentage of essence in the formulation is between 2% and 25% by weight.

4. The perfume formulation according to any one of claims 1 to 3, wherein the percentage of alcohol in the formulation is equal to or less than 16% by weight.

5. Use of a perfume formulation according to any one of claims 1 to 4 in a liquid perfume dispenser.

6. The use of a perfume formulation according to claim 5, wherein the liquid perfume dispenser is a natural evaporative diffuser and/or a nebulizing diffuser.

7. A liquid perfume dispenser **characterised in that** it comprises a reservoir open to the atmosphere and containing a perfume formulation according to any one of claims 1 to 4, as well as at least one reed extending from a first end in contact with the liquid perfume formulation contained in the reservoir to a second end in contact with the atmosphere, from where the perfume formulation evaporates and disperses into the atmosphere.
